# FASCICULE DE BREVET EUROPEEN

(11) **EP 3 016 661 B1**
(45) Date de publication et mention de la délivrance du brevet: **10.10.2018**
(21) Numéro de dépôt: 14761691.6
(22) Date de dépôt: 04.07.2014
(51) Int. Cl.: A61K 31/7088, C12N 15/63, A61K 39/395, A61K 39/00, C07K 16/18

(54) **INHIBITION DE LA SYNTHÈSE DE LA PROTÉINE BETA-APP OU DE L'ACTIVITÉ DU PEPTIDE A-BETA DANS LES PLEXUS CHOROÏDES**
HEMMUNG DER SYNTHESE VON BETA-APP ODER DER AKTIVITÄT DES A-BETA-PEPTIDS IM PLEXUS CHOROIDEUS
INHIBITION OF THE SYNTHESIS OF BETA-APP OR OF THE ACTIVITY OF THE A BETA PEPTIDE IN THE CHOROID PLEXUS

(30) Priorité: 04.07.2013 FR 1356551
(43) Date de publication de la demande: 11.05.2016
(73) Titulaire: Centre National de la Recherche Scientifique, 75016 Paris (FR); College de France, 75231 Paris Cedex 05 (FR); Institut National de la Santé et de la Recherche Médicale (INSERM), 75013 Paris (FR)
(72) Inventeur: DI NARDO, Ariel, F-91120 Palaiseau (FR); MOYA, Kenneth Lee, F-75010 Paris (FR); ARNAUD, Karen, F-75015 Paris (FR); PROCHIANTZ, Alain, F-75006 Paris (FR)
(74) Mandataire: Gevers & Orès
(86) Numéro de dépôt international: PCT/IB2014/062871
(87) Numéro de publication internationale: WO 2015/001532

(56) Documents cités:
- WO-A2-2006/118959
- WO-A2-2009/122401
- US-A1- 2011 081 717
- FUKUCHI K I ET AL: "Anti-Abeta single-chain antibody delivery via adeno-associated virus for treatment of Alzheimer's disease", NEUROBIOLOGY OF DISEASE, BLACKWELL SCIENTIFIC PUBLICATIONS, OXFORD, GB, vol. 23, no. 3, 1 septembre 2006 (2006-09-01), pages 502-511, XP024901519, ISSN: 0969-9961, DOI: 10.1016/J.NBD.2006.04.012 [extrait le 2006-09-01]
- VITE C H ET AL: "Adeno-Associated Virus Vector-Mediated Transduction in the Cat Brain", GENE THERAPY, NATURE PUBLISHING GROUP, GB, vol. 10, 1 janvier 2003 (2003-01-01), pages 1874-1881, XP003002610, ISSN: 0969-7128
- PIAO WENYING; NISHINA KAZUTAKA; YOSHIDA-TANAKA KIE; KUWAHARA HIROYA; NISHINA TOMOKO; SAKATA MINA; MIZUSAWA HIDEHIRO; YOKOTA TAKANO: "Efficient in vivo delivery of antisense oligonucleotide to choroid plexus.", JOURNAL OF MEDICAL AND DENTAL SCIENCES, vol. 60, no. 1, 1 mars 2013 (2013-03-01), pages 9-16, XP55150654, JP ISSN: 1342-8810
- Shinya Shimiu: "Routes of administration" In: "The Laboratory Mouse", 1 janvier 2004 (2004-01-01), Elsevier, XP55150715, pages 527-541, page 535
- ZHI-DONG ZHOU ET AL: "The roles of amyloid precursor protein (APP) in neurogenesis", CELL ADHESION & MIGRATION, vol. 5, no. 4, 1 juillet 2011 (2011-07-01) , pages 280-292, XP55150875, ISSN: 1933-6918, DOI: 10.4161/cam.5.4.16986
- JANELLE S CROSSGROVE ET AL: "The Choroid Plexus Removes b-Amyloid from Brain Cerebrospinal Fluid", EXPERIMENTAL BIOLOGY AND MEDICINE, vol. 230, no. 10, 1 novembre 2005 (2005-11-01), pages 771-776, XP055095165,
- Emily Van Uden ET AL: "Increased Extracellular Amyloid Deposition and Neurodegeneration in Human Amyloid Precursor Protein Transgenic Mice Deficient in Receptor-Associated Protein", Journal of Neuroscience, 1 novembre 2002 (2002-11-01), pages 9298-9304, XP055095152, United States Extrait de l'Internet: URL:http://www.jneurosci.org/cgi/content/a bstract/22/21/9298
- MARTA BOLOS ET AL: "Neurogenic effects of [beta]-amyloid in the choroid plexus epithelial cells in Alzheimer's disease", CELLULAR AND MOLECULAR LIFE SCIENCES, vol. 70, no. 15, 3 mars 2013 (2013-03-03), pages 2787-2797, XP055095154, ISSN: 1420-682X, DOI: 10.1007/s00018-013-1300-x

### SEQUENCE LISTING

<110> CENTRE NATIONAL DE LA RECHERCHE SCIENTIFIQUE COLLEGE DE FRANCE INSTITUT NATIONAL DE LA SANTE ET DE LA RECHERCHE MEDICALE DI NARDO, Ariel MOYA, Kenneth Lee ARNAUD, Karen PROCHIANTZ, Alain
<120> INHIBITION DE LA SYNTHESE DE LA PROTEINE BETA-APP OU DE L'ACTIVITE DU PEPTIDE A-BETA DANS LES PLEXUS CHOROIDES
<130> F644PCT354
<160> 2
<170> PatentIn version 3.5
<210> 1
   <211> 21
   <212> RNA
   <213> artificial sequence
<220>
   <223> synthetic oligoribonucleotide
<400> 1
   augaacuuca uauccugagu c 21
<210> 2
   <211> 21
   <212> DNA
   <213> artificial sequence
<220>
   <223> synthetic oligonucleotide
<400> 2
   gactcaggat atgaagttca t 21

## Description

L'invention est relative à des moyens de restaurer la fonction physiologique du précurseur de la protéine amyloïde (βAPP) et de diminuer la production de peptide β-amyloïde, utilisables dans le cadre de la prévention ou du traitement de maladies neurodégénératives, notamment de la maladie d'Alzheimer.

Le peptide β-amyloïde (peptide Aβ) est le constituant majeur des dépôts amyloïdes extracellulaires observés dans le cortex cérébral des patients atteints de maladie d'Alzheimer. Ce peptide provient du clivage de la protéine transmembranaire βAPP (pour « β-Amyloid Precursor Protein »). Il est important de rappeler que la seule source de peptide Aβ est la protéine βAPP et qu'une réduction de la protéine βAPP entraine ipso-facto une réduction de la production du peptide Aβ. Il faut aussi rappeler que la protéine βAPP a des fonctions physiologiques et que l'une d'entre elles est de réguler la neurogénèse adulte (CAILLE et al., Development, 131, 2173-81, 2004).

Le peptide Aβ résulte de la maturation de la protéine βAPP par la voie dite « amyloïdogénique », impliquant l'action successive de deux activités protéasiques, la β-sécrétase et la γ-sécrétase, qui libèrent respectivement les extrémités N- et C-terminales du peptide.

L'accumulation de peptides Aβ dans le milieu extracellulaire induit des altérations des membranes cellulaires provoquant une entrée massive de calcium dans la cellule et s'accompagnant d'une réaction inflammatoire. Ces lésions entraîneraient la mort neuronale.

Une voie alternative de maturation protéolytique de la protéine βAPP est la voie dite « non amyloïdogénique » ; dans cette voie, une autre enzyme, l'α-sécrétase, coupe la βAPP au milieu de la séquence Aβ. Cette coupure prévient la formation du peptide Aβ, et libère une forme soluble et sécrétée dénommée sAPP, qui a des propriétés neuroprotectrices et une fonction mitogène sur les cellules souches neurales adultes (CAILLE et al., Development, 131, 2173-81, 2004). La production du sAPP est donc incompatible avec celle du βA4 généré par l'action de la béta- (en N-terminal) et la gamma- (en C-terminal) secrétase.

La protéine βAPP est exprimée dans de nombreux tissus de l'organisme, et notamment par les neurones, y compris au niveau cérébral.

Les plexus choroïdes sont des structures localisées au niveau des ventricules, plus particulièrement au niveau du toit du 4^{e} ventricule et de la jonction entre les ventricules latéraux du cerveau et le 3^{e} ventricule. Ils sont constitués d'un épithélium constitué de cellules épendymaires associées par des jonctions serrées et reposant sur une membrane basale qui les sépare d'un stroma conjonctivo-vasculaire composé d'un réseau de capillaires sanguins fenestrés, et de fibres de collagène produites par les fibroblastes présents dans ce stroma.

Les plexus choroïdes fabriquent le liquide céphalorachidien (LCR), en jouant un rôle de barrière sélective autorisant le passage de certaines molécules entre le sang et le LCR, et en bloquant d'autres. Ils synthétisent également un certain nombre de protéines, qui sont sécrétées dans le LCR. Bien que la présence de βAPP dans les plexus choroïdes ait été rapportée (SASAKI et al., Brain Res, 755, 193-201, 1997), son niveau d'expression n'avait pas été déterminé auparavant.

Les Inventeurs ont maintenant constaté, lors d'une analyse par séquençage des ARN des cellules épithéliales du plexus choroïde, que le gène codant pour βAPP faisait partie de ceux qui étaient les plus fortement exprimés dans cette structure, et que le niveau d'expression de cette protéine dans le plexus choroïde était très supérieur à celui observé dans d'autres régions du cerveau précédemment connues pour l'exprimer à un niveau élevé. Ils ont également observé une expression très élevée de la protéine APLP-2, qui appartient à la même famille que βAPP et qui comme celle-ci peut générer par maturation protéolytique une forme soluble (sAPLP-2) ayant des propriétés neuroprotectrices et neurotrophes, mais qui, contrairement à βAPP ne forme pas de peptide Aβ car l'APLP-2 ne contient pas la séquence Aβ (WASCO et al., Proc Natl Acad Sci U S A, 89, 10758-62, 1992).

Cette observation faite par les Inventeurs de l'importance quantitative des plexus choroïdes comme source de βAPP permet de proposer de cibler cette structure cérébrale pour y réguler la production de βAPP, soit en l'augmentant, soit en la diminuant. Il est clair que diminuer l'expression de βAPP dans les plexus choroïdes doit diminuer celle du peptide Aβ. Cette structure peut aussi être ciblée pour des stratégies visant à bloquer ou diminuer l'activité du peptide Aβ.

La présente invention concerne le ciblage du plexus choroïde dans des stratégies de régulation de la synthèse de la protéine βAPP ou d'adressage d'un inhibiteur de l'activité du peptide Aβ, dans le cadre du traitement d'une maladie neurodégénérative.

La présente invention a en conséquence pour objet un inhibiteur de la synthèse de la protéine βAPP ou de l'activité du peptide Aβ ou un vecteur d'expression codant pour ledit inhibiteur, pour son utilisation dans le traitement d'une maladie neurodégénérative qui est une forme sporadique ou familiale de la maladie d'Alzheimer, par ciblage dudit inhibiteur dans les plexus choroïdes pour y diminuer la production ou l'activité dudit peptide Aβ, ledit inhibiteur étant choisi parmi les oligonucléotides antisens et les ARNs interférents dirigés contre le gène codant pour ladite protéine beta-APP.

Les fonctionnalités de la protéine βAPP liées à la production de sAPP peuvent être compensées par la protéine APLP-2, qui est également fortement exprimée dans les plexus choroïdes.

Si cette compensation n'est pas suffisante, on peut surexprimer le sAPP sous forme recombinante dans les cellules épithéliales des plexus choroïdes. Dans ce cas, l'oligonucléotide antisens ou l'ARNi utilisé pour inhiber la synthèse de la protéine βAPP sera préférentiellement dirigé contre une région du gène codant pour la partie C-terminale de cette protéine, afin de ne pas interférer avec la synthèse du sAPP recombinant.

Si l'on choisit d'inhiber l'activité du peptide Aβ, on peut utiliser dans ce but un anticorps ou un fragment d'anticorps sélectivement dirigé contre ce peptide et capable de bloquer son activité. De nombreux anticorps possédant cette propriété sont connus en eux-mêmes ; à titre d'exemples non-limitatifs, on citera scFV Aβ^{IB}, scFVAβ_{KDE}^{IB} (SUDOL et al., Mol Ther, 17, 2031-40, 2009), scFV59 (FUKUCHI et al., Neurobiol Dis, 23, 502-11, 2006), ou CBAβ42 (ZHANG et al., Neurobiol Dis, 14, 365-79, 2003).

Des vecteurs utilisables dans le cadre de la présente invention pour l'expression des oligonucléotides antisens, des ARNi dirigés contre le peptide Aβ, dans les cellules du plexus choroïde sont des vecteurs viraux ciblant préférentiellement ces cellules. Il s'agit par exemple de vecteurs dérivés de virus adéno-associés (AAV) des sérotypes 2, 4 ou 5 (CACHON-GONZALEZ et al., Mol Ther, 20, 1489-500, 2012; DODGE et al., Mol Ther, 18, 2075-84, 2010; DONSANTE et al., Mol Ther, 19, 2114-23, 2011; WATSON et al., Hum Gene Ther, 16, 49-56, 2005).

Quel que soit le vecteur choisi, on peut également placer l'oligonucléotide antisens, l'ARNi dirigé contre le peptide Aβ, sera placé dans le vecteur choisi, sous contrôle d'un promoteur spécifique des cellules du plexus choroïde ; à titre d'exemples non-limitatifs de promoteurs utilisables dans ce cadre, on citera le promoteur du gène CRFR2β (REGEV et al., Proc Natl Acad Sci USA, 107, 4424-9, 2010), le promoteur de la transthyrétine (COSTA et al., Molecular and Cellular Biology, 6, 4697-708, 1986), ou celui du gène GPR125 (PICKERING et al., BMC Neuroscience, 9, 97, 2008).

Le plexus choroïde a aussi l'avantage d'être facilement accessible par des voies peu invasives, en particulier via l'injection de substances pharmacologiques dans le système veineux et, plus favorablement, dans le sinus rétro-orbital qui est tout proche de la face baso-latérale du plexus choroïde. Il est donc possible d'adresser au plexus choroïde des molécules ou des vecteurs viraux pouvant modifier transitoirement ou de façon permanente l'expression et/ou la sécrétion du βAPP, par le plexus choroïde.

Egalement décrit est le traitement d'une maladie neurodégénérative, comprenant le ciblage dans les plexus choroïdes d'un patient, d'une quantité efficace d'un inhibiteur de la synthèse de la protéine βAPP ou de l'activité du peptide Aβ ou d'un vecteur d'expression codant pour ledit inhibiteur, pour diminuer la production ou l'activité dudit peptide Aβ dans les plexux choroïdes dudit patient.

Ladite méthode peut comprendre également le ciblage dans les plexus choroïdes dudit patient, d'une quantité efficace d'une forme soluble fonctionnelle de la protéine βAPP, pour y restaurer ou augmenter une fonction physiologique de ladite protéine βAPP, notamment la neurogénèse. La forme soluble fonctionnelle de la protéine βAPP est une protéine soluble dérivée de la protéine βAPP qui conserve les fonctions physiologiques de la protéine βAPP; il s'agit notamment de la sAPP.

Egalement décrite est une préparation combinée comprenant :
(i) un inhibiteur de la synthèse de la protéine βAPP ou de l'activité du peptide Aβ ou un vecteur d'expression codant pour ledit inhibiteur et
(ii) une forme soluble fonctionnelle de la protéine βAPP ou un vecteur d'expression codant pour ladite forme soluble,
pour une utilisation simultanée, séparée ou séquentielle dans le traitement d'une maladie neurodégénérative, par ciblage des plexus choroïdes du patient pour y diminuer la production ou l'activité dudit peptide Aβ et restaurer ou augmenter une fonction physiologique de ladite protéine βAPP, notamment la neurogénèse.

Les maladies neurodégénératives pouvant être traitées conformément à l'invention sont notamment les formes sporadiques et familiales de la maladie d'Alzheimer.

La présente invention sera mieux comprise à l'aide du complément de description qui va suivre, qui se réfère à des exemples non-limitatifs démontrant l'expression de βAPP dans les plexus choroïdes, et décrivant la construction de vecteurs viraux permettant de réguler cette expression ou d'inhiber l'activité du peptide βA4, ainsi qu'aux dessins annexés dans lesquels :

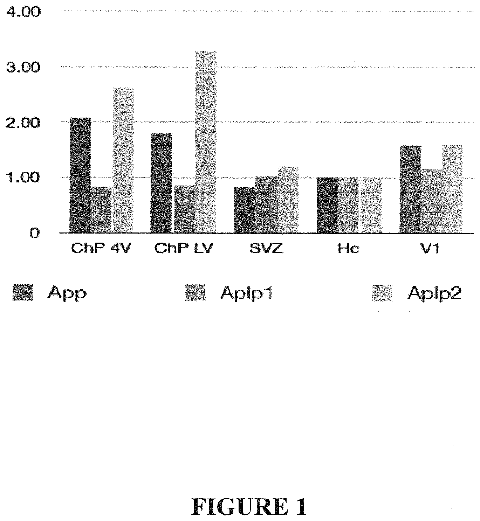

- la figure 1 représente les niveaux relatifs des ARNm des gènes App, Aplp1 et Aplp2 rapportés à ceux du gène de ménage HPRT, dans le 4ème ventricule (ChP 4V), les ventricules latéraux (ChP LV), l'hippocampe (Hc), la zone subventriculaire (SVZ)et le cortex visuel primaire (V1) de souris âgées d'environs 8 semaines.

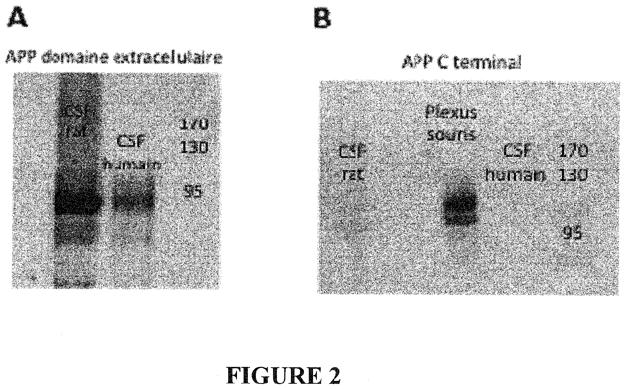

- la figure 2 illustre la détection des formes transmembranaires et sécrétées de la protéine βAPP dans le liquide céphalo-rachidien et le plexus choroïde. **A.** Western Blot avec un anticorps dirigé contre la partie N-terminale de la protéine βAPP (APP domaine extracellulaire), spécifique de la forme sécrétée. **B.** Western Blot avec un anticorps dirigé contre la partie C-terminale de la protéine βAPP (APP C terminal), spécifique de la forme transmembranaire. CSFrat : liquide céphalo-rachidien de rat. CSFh : liquide céphalo-rachidien humain. Plexus mouse : plexus choroïde de souris.

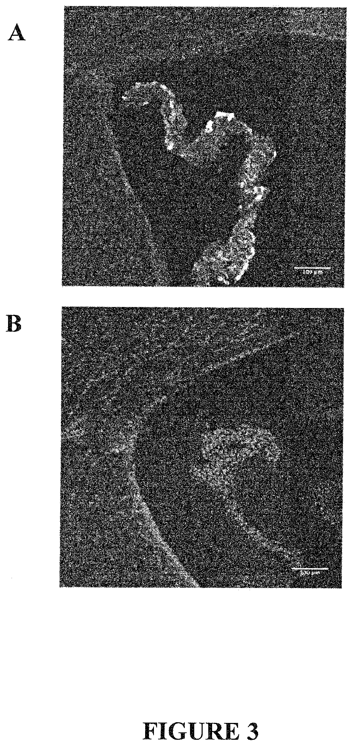

- la figure 3 illustre le ciblage spécifique des plexus choroïdes par l'AAV de sérotype 5 (AAV5). L'activité GFP du cerveau de souris porteuses du gène encodant la GFP mais ne l'exprimant qu'après recombinaison, a été analysée après injection dans les ventricules cérébraux, d'un AAV5 porteur ou non du gène codant pour la recombinase CRE. **A.** AAV5-CRE. **B.** AAV5 contrôle.

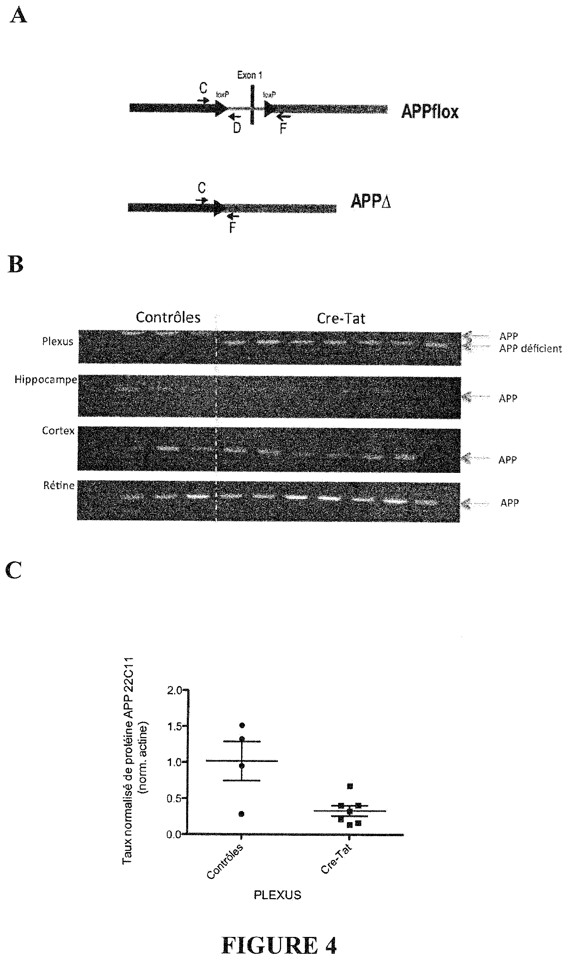

- la figure 4 illustre la recombinaison du gène βAPP dans le plexus choroïde. **A.** Représentation schématique du gène βAPP non-recombiné (APPflox) et recombiné par la recombinase CRE (APPΔ) dans la lignée de souris APP^{flox/flox}. Des souris APP^{flox/flox} ont été injectées dans les ventricules cérébraux avec la protéine CRE-Tat ou un véhicule à titre de contrôle. 15 jours après l'injection, l'ADN génomique du plexus choroïde (plexus), de l'Hippocampe (Hippocampus), du Cortex cérébral (Cortex) et de la rétine (Retina)a été analysé par PCR **(B)** et la quantité de protéine βAPP dans le plexus choroïde a été quantifiée par Western blot **(C).**

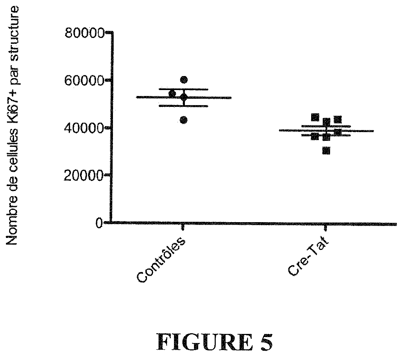

- la figure 5 illustre la diminution du nombre de cellules prolifératives (Ki67+) dans la zone sous ventriculaire (SVZ), après diminution de la protéine βAPP dans le plexus choroïde par recombinaison génétique du gène βAPP.

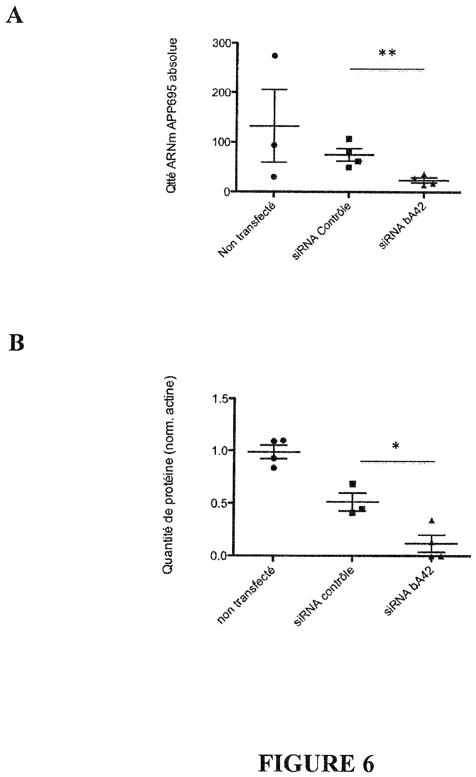

- la figure 6 illustre la diminution d'ARNm βAPP (A) et de protéine βAPP (B) dans les cellules Hela transfectées avec un ARNi ciblant le peptide Aβ.

### EXEMPLE 1 : EXPRESSION DE LA PROTÉINE βAPP DANS LES PLEXUS CHOROÏDES.

Nous avons comparé le niveau d'expression de βAPP et de deux molécules proches, APLP-1 et APLP-2, dans le plexus choroïde, l'hippocampe, la zone subventriculaire et le cortex visuel primaire d'une souris d'environ 8 semaines d'âge.

Après dissection des tissus, les ARNs ont été extraits et l'abondance des ARNs messagers pour les gènes APP, APLP1 et APLP2 déterminés par RT-qPCR grâce à des amorces spécifiques de chaque gène. Les niveaux d'expression sont rapportés à celui d'un gène de ménage, l'HPRT. Les résultats sont illustrés par la Figure 1 et le Tableau I ci-dessous.

Légende de la Figure 1 et du Tableau I : 4ème ventricule : ChP 4V ; ventricules latéraux : ChP LV ; hippocampe : Hc) ; zone subventriculaire : SVZ ; cortex visuel primaire : V1.

**Tableau I**

| | **App** | **Aplp1** | **Aplp2** |
|---|---|---|---|
| **ChP 4V** | 2.07 | 0.82 | 2.61 |
| **ChP LV** | 1.80 | 0.86 | 3.28 |
| **SVZ** | 0.82 | 1.03 | 1.19 |
| **Hc** | 1.00 | 1.00 | 1.00 |
| **V1** | 1.58 | 1.15 | 1.59 |

Ces résultats montrent clairement que le plexus choroïde exprime 2 fois à 3 fois plus de transcrits d'APP et d'APLP2 que les autres structures.

### EXEMPLE 2 : LA βAPP ENTIÈRE EST PRÉSENTE DANS LE PLEXUS CHOROIDE ET SA FORME SOLUBLE EST SECRETÉE DANS LE LCR

Le plexus choroïde d'un rongeur a été analysé par Western blot avec un anticorps dirigé contre la partie C-terminale de la βAPP (Figure 2B). L'anticorps révèle un signal à 105 et 120kDa correspondant à la forme entière et transmembranaire du βAPP. La présence de βAPP secrétée (sAPP) dans le liquide céphalo-rachidien (LCR) de rongeur et d'humain a été recherchée par Western blot avec un anticorps dirigé contre la partie N-terminale de la protéine (donc extracellulaire et potentiellement secrétée après clivage par l'alpha-sécrétase). L'anticorps révèle un signal vers 90kDa correspondant à la forme clivée et secrétée du βAPP (sAPP) dans le LCR (Figure 2A).

### EXEMPLE 3 : L'AAV DE SÉROTYPE 5 (AAV5) CIBLE SPÉCIFIQUEMENT LES PLEXUS CHOROÏDES POUR LA RECOMBINASION GENETIQUE

Auparavant nous avons montré que la protéine de fusion de la recombinase CRE avec le vecteur peptidique dérivé de la protéine TAT du VIH (CRE-TAT) injectée dans les ventricules cérébraux de souris adultes est capable d'induire une recombinaison génomique spécifique dans le plexus choroïde (Spatazza et al., Cell Reports 3 : 1815-1823, 2013).

Afin de démontrer qu'un vecteur viral cible spécifiquement le plexus choroïde, un AAV5 porteur ou non du gène codant pour la recombinase CRE a été injecté dans les ventricules cérébraux de souris porteuses du gène encodant la GFP mais ne l'exprimant qu'après recombinaison par la recombinase CRE. La Figure 3A démontre que le virus a accès au plexus choroïde mais n'infecte pas le parenchyme avoisinant et que la CRE exprimée par le génome viral induit l'expression de la GFP dans une grande quantité de cellules du plexus choroïde.

### EXEMPLE 4 : LE GÈNE βAPP PEUT ETRE RECOMBINE DANS LE PLEXUS CHOROIDE

Des souris APP^{flox/flox} adulte (Mallim et al., Genesis 48 : 200-206, 2010 ; Figure 4A ont été injectées avec le véhicule ou la protéine CRE-TAT dans les ventricules cérébraux. Quinze jours plus tard, l'ADN génomique a été extrait de différentes régions du cerveau et analysé par PCR. Le gel de la Figure 4B démontre que le gène du βAPP a été recombiné de façon spécifique dans le plexus choroïde, à l'exclusion d'autres structures du système nerveux central comme la rétine, le néocortex ou l'hippocampe. L'analyse par western blot confirme que la quantité de protéine βAPP est diminuée de façon significative dans le plexus choroïde 15 jours après recombinaison (Figure 4C).

### EXEMPLE 5 : LA DIMINUTION DU BAPP DANS LE PLEXUS CHOROIDE ENTRAINE UNE RÉDUCTION DE NOMBRE DE CELLULES PROLIFERATIVES DANS LA ZONE SOUS VENTRICULAIRE.

Les cellules prolifératives des souris dans lesquelles le gène βAPP a été recombiné (exemple 4) ont été identifiées grâce à un anticorps dirigé contre la protéine Ki67 (marqueur de prolifération) et puis comptées par stéréologie. Par rapport aux souris contrôles, les souris dans lesquelles le gène βAPP a été recombiné voient leur neurogénèse (nombre de cellules prolifératives) diminuée de façon significative (Figure 5).

### EXEMPLE 6: LA sAPP EXOGENE INJECTÉE DANS LES VENTRICULES CEREBRAUX AUGMENTE LA NEUROGENESE DANS LE SVZ.

La sAPP recombinante a été injectée dans les ventricules cérébraux des souris sauvages adultes de six semaines. Une et trois semaines plus tard les cellules prolifératives sont identifiées grâce au marqueur Ki67 comme dans l'exemple 5. Une augmentation du nombre des cellules prolifératives est attendue.

### EXEMPLE 7 : CONSTRUCTION D'UN VECTEUR VIRAL POUR L'EXPRESSION DE sAPP DANS LE PLEXUS CHOROIDE.

La séquence codante pour sAPP (nt 201-2213 de NM_000484, Genbank) est insérée dans un vecteur lentiviral dérivé de pTRIPΔU3[PGK+beta2/IRES2+EGFP+WPRE] (MASKOS et al., 2005, Nature 436: 103-107), en aval du promoteur FoxJ1 qui n'est pas actif dans les cellules neuronales (ZHANG et al., 2007, Am J Respir Cell Mol Biol 36: 515-519), et en amont de la séquence codante d'un peptide intermédiaire (P2A, KIM et al., 2011, PLoS one 6(4) e18556) suivie de celle de la GFP.

Le plasmide résultant (pLFsAPP-P2Gfp) est co-transfecté avec les plasmides codant pour les protéines virales requises dans une lignée d'empaquetage appropriée (HEK 293T). Les particules virales sont ensuite récupérées dans le milieu de culture, concentrées et titrées.

Les lentivirus obtenus sont injectés dans le sinus retro-orbital, d'où ils ont un accès privilégié au plexus choroïde.

### EXEMPLE 8: CONSTRUCTION D'UN VECTEUR POUR L'EXPRESSION D'UN ANTICORPS À SIMPLE CHAÎNE CONTRE LE βA4

Les ARNm extraits d'hybridomes exprimant un anticorps monoclonal anti-βA4 tel que scFV Aβ^{IB}, scFVAβ_{KDE}^{IB} (SUDOL et al., 2009, précité), scFV59 (FUKUCHI et al., 2006, précité) ou CBAβ42 (ZHANG et al., 2003, précité), sont utilisés pour amplifier séparément les parties variables des chaines lourdes et légères selon le protocole décrit par BARBAS et al. (2001, "Phage display, a laboratory manual", CSHL Press) adapté dans le laboratoire (LESAFFRE et al., Neural Development, 2, 2, 2007). Disposés de part et d'autre de la séquence codante d'un long lieur flexible, ces minigènes sont fusionnés à une étiquette formée de 6 tag myc, et introduits dans le vecteur lentiviral bi-cistronique décrit ci-dessus (Cf. Exemple 7).

Le plasmide résultant (pLFsabA4-P2Gfp) est utilisé pour produire des lentivirus qui sont injectés comme décrit ci-dessus (cf. Exemple 7).

### EXEMPLE 9: CONSTRUCTION D'UN PETIT ARN INTERFERENT POUR DIMINUER L'EXPRESSION DU GÈNE βAPP ET DE LA QUANTITE DE PROTEINE βAPP.

Un petit ARN interférent (ARNi ou siRNA) a été préparé ; sa séquence 5'-AUGAACUUCAUAUCCUGAGTC-3'(SEQ ID NO : 1) complémentaire de la séquence 5'-GACTCAGGATATGAAGTTCAT-3' (SEQ ID NO : 2) est spécifique du domaine ADN codant pour une partie du peptide Aβ humain. Les cellules humaines de lignée HeLa sont mises en culture et 24h plus tard un siARN contrôle (i.e., correspondant à aucune séquence humaine dans la base de donnés BLAST) ou l'ARNi a été transfecté (100pmol/100.000cellules). 48 heures plus tard les cellules ont été lysées et le taux d'ARNm βAPP analysé par RT-qPCR et la quantité de protéine βAPP par Western blot. Après transfection avec l'ARNi le taux d'ARNm βAPP est diminué de façon significative (par rapport aux cellules transfectées avec le siRNA contrôle),(Figure 6A). La quantité de protéine βAPP est aussi diminuée de façon significative dans les cellules transfectées avec l'ARNi(Figure 6B).

### EXEMPLE 10: UTILISATION D'UN AAV5 EXPRIMANT UN PETIT ARN INTERFERENT POUR DIMINUER L'EXPRESSION DE βAPP DANS LE PLEXUS CHOROIDE ET RETARDER LA FORMATION DE PLAQUES SENILES DANS LE CERVEAU DES SOURIS APP/PS1.

Un AAV5 exprimant le shARN correspondant au petit ARNi de l'exemple 9 sous le contrôle du promoteur U6 a été construit. Les souris portant une mutation « Swedish » dans le gène codant pour la βAPP et portant une mutation dans le gène codant pour le PS1 développent des agrégats Aβ (plaques séniles) dans l'hippocampe et le néocortex à partir de 4 mois. Ces souris sont injectées avec l'AAV5-shARN dans les ventricules cérébraux entre 3 et 5 mois et à 6 mois le nombre et la taille des dépôts Aβ sont identifiés par marquage Thioflavin S et des anticorps anti-Aβ sont analysés. Une diminution dans le nombre de plaques est attendue.

## Revendications

1. Inhibiteur de la synthèse de la protéine βAPP ou de l'activité du peptide Aβ ou vecteur d'expression codant pour ledit inhibiteur, pour son utilisation dans le traitement d'une maladie neurodégénérative qui est une forme sporadique ou familiale de la maladie d'Alzheimer, par ciblage dudit inhibiteur ou vecteur dans les plexus choroïdes pour y diminuer la production dudit peptide Aβ,
ledit inhibiteur étant choisi parmi les oligonucléotides antisens et les ARNs interférents dirigés contre le gène codant pour ladite protéine βAPP.

2. Vecteur codant pour un inhibiteur de la synthèse de la protéine βAPP ou de l'activité du peptide Aβ, pour son utilisation dans le traitement d'une maladie neurodégénérative selon la revendication 1, **caractérisé en ce que** ledit vecteur est un vecteur viral permettant l'expression spécifique dudit inhibiteur dans les cellules des plexus choroïdes.

3. Vecteur viral codant pour un inhibiteur de la synthèse de la protéine βAPP ou de l'activité du peptide Aβ pour son utilisation dans le traitement d'une maladie neurodégénérative selon la revendication 2, **caractérisé en ce qu'**il s'agit d'un vecteur dérivé d'un virus adéno-associé de sérotype 2, 4 ou 5.

4. Vecteur viral codant pour un inhibiteur de la synthèse de la protéine βAPP ou de l'activité du peptide Aβ pour son utilisation dans le traitement d'une maladie neurodégénérative selon la revendication 2 ou 3, **caractérisé en ce que** ledit inhibiteur est sous contrôle d'un promoteur spécifique des cellules du plexus choroïde.

5. Inhibiteur de la synthèse de la protéine βAPP ou de l'activité du peptide Aβ ou vecteur d'expression codant pour ledit inhibiteur, pour son utilisation dans le traitement d'une maladie neurodégénérative selon l'une quelconque des revendications 1 à 4, **caractérisé en ce que** ledit inhibiteur ou vecteur est administré par injection dans le sinus rétro-orbital.

6. Inhibiteur de la synthèse de la protéine βAPP ou de l'activité du peptide Aβ ou vecteur d'expression codant pour ledit inhibiteur, pour son utilisation dans le traitement d'une maladie neurodégénérative selon l'une quelconque des revendications 1 à 5, **caractérisé en ce que** ledit inhibiteur ou vecteur est combiné à une forme soluble fonctionnelle de la protéine βAPP.

## Patentansprüche

1. Inhibitor der Synthese des Proteins βAPP oder der Aktivität des Peptids Aβ, oder Expressionsvektor, der für den Inhibitor kodiert, zur Verwendung bei der Behandlung einer neurodegenerativen Krankheit, die eine sporadische oder familiäre Form der Alzheimerkrankheit ist, durch Targeting des Inhibitors oder Vektors in den Plexus choroidei, um dort die Produktion des Peptids Aβ zu verringern,
wobei der Inhibitor ausgewählt ist aus den Antisense-Oligonukleotiden und den gegen das Gen, das für das Protein βAPP kodiert, gerichteten interferierenden RNAs.

2. Vektor, der für einen Inhibitor der Synthese des Proteins βAPP oder der Aktivität des Peptids Aβ kodiert, zur Verwendung bei der Behandlung einer neurodegenerativen Krankheit nach Anspruch 1, **dadurch gekennzeichnet, dass** der Vektor ein viraler Vektor ist, der die spezifische Expression des Inhibitors in den Zellen der Plexus choroidei erlaubt.

3. Viraler Vektor, der für einen Inhibitor der Synthese des Proteins βAPP oder der Aktivität des Peptids Aβ kodiert, zur Verwendung bei der Behandlung einer neurodegenerativen Krankheit nach Anspruch 2, **dadurch gekennzeichnet, dass** es sich um einen von einem adenoassoziierten Virus des Serotyps 2, 4 oder 5 abgeleiteten Vektor handelt.

4. Viraler Vektor, der für einen Inhibitor der Synthese des Proteins βAPP oder der Aktivität des Peptids Aβ kodiert, zur Verwendung bei der Behandlung einer neurodegenerativen Krankheit nach Anspruch 2 oder 3, **dadurch gekennzeichnet, dass** der Inhibitor unter der Kontrolle eines spezifischen Promoters von Zellen des Plexus choroideus steht.

5. Inhibitor der Synthese des Proteins βAPP oder der Aktivität des Peptids Aβ, oder Expressionsvektor, der für den Inhibitor kodiert, zur Verwendung bei der Behandlung einer neurodegenerativen Krankheit nach einem der Ansprüche 1 bis 4, **dadurch gekennzeichnet, dass** der Inhibitor oder Vektor durch Injektion in den retrobulbären Venenplexus verabreicht wird.

6. Inhibitor der Synthese des Proteins βAPP oder der Aktivität des Peptids Aβ, oder Expressionsvektor, der für den Inhibitor kodiert, zur Verwendung bei der Behandlung einer neurodegenerativen Krankheit nach einem der Ansprüche 1 bis 5, **dadurch gekennzeichnet, dass** der Inhibitor oder Vektor kombiniert wird mit einer löslichen funktionellen Form des Proteins βAPP.

## Claims

1. Inhibitor of the synthesis of the βAPP protein or of the activity of the Aβ peptide or the expression vector coding for said inhibitor, for its use in the treatment of a neurogenerative disease that is a sporadic or familial form of Alzheimer's disease, by targeting said inhibitor or vector in the choroid plexuses to reduce in them the production of said Aβ peptide,
said inhibitor being chosen from among antisense oligonucleotides and interfering RNAs directed against the gene coding for said βAPP protein.

2. Vector coding for an inhibitor of the synthesis of the βAPP protein or of the activity of the Aβ peptide, for its use in the treatment of a neurogenerative disease according to claim 1, **characterised in that** said vector is a viral vector enabling the specific expression of said inhibitor in the choroid plexus cells.

3. Viral vector coding for an inhibitor of the synthesis of the βAPP protein or of the activity of the Aβ peptide, for its use in the treatment of a neurogenerative disease according to claim 2, **characterised in that** it is a vector derived from an adeno-associated virus of serotype 2, 4 or 5.

4. Viral vector coding for an inhibitor of the synthesis of the βAPP protein or of the activity of the Aβ peptide, for its use in the treatment of a neurogenerative disease according to claim 2 or 3, **characterised in that** said inhibitor is under control of a specific promotor of choroid plexus cells.

5. Inhibitor of the synthesis of the βAPP protein or of the activity of the Aβ peptide or the expression vector coding for said inhibitor, for its use in the treatment of a neurogenerative disease according to any one of claims 1 to 4, **characterised in that** said inhibitor or vector is administered by injection in the retro-orbital sinus.

6. Inhibitor of the synthesis of the βAPP protein or of the activity of the Aβ peptide or the expression vector coding for said inhibitor, for its use in the treatment of a neurogenerative disease according to any one of claims 1 to 5, **characterised in that** said inhibitor or vector is combined to a functional soluble form of the βAPP protein.
